# EUROPEAN PATENT SPECIFICATION

(11) **EP 2 732 026 B1**
(45) Date of publication and mention of the grant of the patent: **30.03.2016**
(21) Application number: 12705996.2
(22) Date of filing: 08.02.2012
(51) Int. Cl.: C12N 1/20, C12R 1/01, C12R 1/125, C12R 1/40, B09C 1/10, C02F 3/34

(54) **BIOLOGICAL PRODUCT FOR CLEARING OF WATER, INDUSTRIAL WASTEWATER AND SOIL FROM CHEMICALS, WHICH ARE RESISTANT TO DEGRADATION AND METHOD FOR USING THE SAME**
BIOLOGISCHES PRODUKT ZUR REINIGUNG VON WASSER, INDUSTRIELLEN ABWÄSSERN UND BÖDEN VON ZERSETZUNGSRESISTENTEN CHEMIKALIEN UND VERWENDUNGSVERFAHREN DAFÜR
PRODUIT BIOLOGIQUE POUR L'ÉPURATION DE L'EAU, DES EAUX USÉES INDUSTRIELLES ET DE TERRE EN ÉLIMINANT DES PRODUITS CHIMIQUES, QUI SONT RÉSISTANTS À LA DÉGRADATION ET SON PROCÉDÉ D'UTILISATION

(30) Priority: 13.07.2011 RU 2011128996
(43) Date of publication of application: 21.05.2014
(73) Proprietor: Bioland, Ltd, Moskva 117292 (RU); Zakutajeva, Galina, 1013 Riga (LV)
(72) Inventor: ANISIMOVA, Liliya, Ufa 450069 Republic of Bashkortostan (RU); MARKUSHEVA, Tatyana, Ufa 450069 Republic of Bashkortostan (RU); KURAKOV, Aleksandr, Moskva 125222 (RU); ZAKUTAYEV, Andrey, Moskva 119048 (RU)
(74) Representative: Fortuna, Jevgenijs
(86) International application number: PCT/EP2012/052089
(87) International publication number: WO 2013/007398

(56) References cited:
- DATABASE CA [Online] CHEMICAL ABSTRACTS SERVICE, COLUMBUS, OHIO, US; 2006, Khalimova, L Kh et al: "Study of degradation of phenol, chlorophenols, and 2,4-dichlorophenoxyacetic acid by a consortium of microorganisms (destructors)", XP002674651, retrieved from STN Database accession no. 146:234690 -& KHALIMOVA, L. KH. ET AL: "Study of degradation of phenol, chlorophenols, and 2,4-dichlorophenoxyacetic acid by a consortium of microorganisms (destructors)", BASHKIRSKII KHIMICHESKII ZHURNAL, vol. 13, no. 1, 2006, pages 71-73, XP009158603, ISSN: 0869-8406
- DATABASE CA [Online] CHEMICAL ABSTRACTS SERVICE, COLUMBUS, OHIO, US; 2007, Khalimova, L. Kh et al: "Preparation of active biomass of microorganisms-destructors of phenols and 2,4- dichlorophenoxyacetic acid", XP002674652, retrieved from STN Database accession no. 148:126970 -& KHALIMOVA, L. KH. ET AL: "Preparation of active biomass of microorganisms-destructors of phenols and 2,4-dichlorophenoxyacetic acid", BASHKIRSKII KHIMICHESKII ZHURNAL, vol. 14, no. 1, 2007, pages 172-174, XP009158604, ISSN: 0869-8406
- DATABASE WPI Week 199824 Thomson Scientific, London, GB; AN 1998-270230 XP002674653, & RU 2 093 478 C1 (UNIV UFA PETROLEUM TECHN) 20 October 1997 (1997-10-20) cited in the application
- DATABASE HCA [Online] YAGAFAROVA, G. T. ET AL: "Biotechnological removal of oil and polymeric reagents from drilling wastes", XP002674654, retrieved from STN Database accession no. 131:233080 -& YAGAFAROVA, G. T. ET AL: "Biotechnological removal of oil and polymeric reagents from drilling wastes", PRIKLADNAYA BIOKHIMIYA I MIKROBIOLOGIYA, vol. 35, no. 2, 1999, pages 178-181, XP009158587, ISSN: 0555-1099
- DATABASE EMBL [Online] 2 December 2010 (2010-12-02), "Bacillus subtilis partial 16S rRNA gene, strain ZWQ-1", XP002674655, retrieved from EBI accession no. EM_PRO:FR729926 Database accession no. FR729926
- DATABASE EMBL [Online] 15 July 2006 (2006-07-15), "Pseudomonas sp. ND9 16S ribosomal RNA gene, partial sequence.", XP002674656, retrieved from EBI accession no. EM_PRO:DQ657850 Database accession no. DQ657850
- DATABASE EMBL [Online] 23 December 2010 (2010-12-23), "Rhodococcus sp. BZ4 16S ribosomal RNA gene, partial sequence.", XP002674657, retrieved from EBI accession no. EM_PRO:HQ588862 Database accession no. HQ588862
- BAEK, KYUNG-HWA ET AL: "Detection of Nocardia sp. H17-1 by PCR during bioremediation of crude", HAN'GUK MISAENGMUL-SAENGMYONGKONG HAKHOECHI, vol. 32, no. 1, 2004, pages 91-95, XP009158585, ISSN: 1598-642X
- HUANG L ET AL: "Optimization of nutrient component for diesel oil degradation by Rhodococcus erythropolis", MARINE POLLUTION BULLETIN, vol. 56, no. 10, 1 October 2008 (2008-10-01), pages 1714-1718, XP025469834, OXFORD, GB ISSN: 0025-326X, DOI: 10.1016/J.MARPOLBUL.2008.07.007 [retrieved on 2008-09-07]

## Description

### Technical Field

The invention relates to a novel biological product for purification of water, soil and industrial wastewater contaminated with chemicals resistant to degradation categorized as hazardous, such as the widely used in agriculture pesticides.

### Background Art

Biological preparations - biodestruktors and their use for the treatment of soil and ground from oil and petroleum products containing *Bacillus brevis* and *Arthrobacter species* are described in the patents RU 2323970 and RU 2237711. The patent RU 2086667 describes a consortium comprising the microorganisms *Pseudomonas putida* and *Bacillus subtilis.*

There are also known biological preparations "Roder" based on strains *Rhodococcus ruber* VKM Ac-1513D and *Rhodococcus erythropolis* VKM Ac-1514D, "Lenoil", "Devoroil", "Ekobel" based on strains of bacteria *Pseudomonas* and yeast, which are effectively used to remove aliphatic fractions of oil.

There is known decomposition of chlorinated aromatic pesticides (2,4-dichlorofenoxyacetic acid and 2,4,5-trichlorofenoxyacetic acid) using bacteria *Pseudomonas pseudoalcaligenes* strain NRRL B-18087 (patent US 4804629), detoxification of organophosphorus pesticide residues and their toxic metabolites - by a strain of bacteria *Agrobacterium radiobacter* (author's certificate SU 1250572). Author's Certificate SU 1560487 describes a method for destruction of the pesticide 3,4-dichloranilide using algae *Chlorella vulgaris* BKM-A-10 and *Scenedesmus acuminalus* UA-2-7a. Decomposition of 2-chlorobenzoic acid using bacteria *Pseudomonas putida* YNK-1 is being described in patent US 4803166; phosphate contaminants and chlorinated hydrocarbons - by microorganisms *Moraxella* and *Arthrobacter* alone or in combination - in patent DE 3729127.

There is known degradation of compounds such as DDT, polychlorphenols, benzopyrenes, dioxines using basidiomycete *Phanerochate chrysosporium* (patent US 4891320).

Strain *Pseudomonas putida -* 106 is an active destructor of dimethylphenylcarbinol and phenol [1, 2], the bacteria *Pseudomonas pseudoacaligenes* destroys the aromatic and heterocyclic compounds, most often found in wastewater [3, 4, 5], and the strain of *Pseudomonas pseudoacaligenes* destroys the aromatic compounds in the solid and liquid medium.

Some museum strains of *Nocardia, Arthrobacter, Micromonospora* [7, 8, 9] have capacity for destruction of chlorotriazine derivatives, such as simazine and atrazine.

Patent US 6632363 describes a composition comprising a hydrophobic carrier, and the bacteria *Bacillus subtilis,* and the method of its use for improving the quality of water containing, for example, pesticides.

Ksenofontova O. et al describes as the most active degraders, strains of aerobic bacteria from the genera *Bacillus* and *Pseudomonas,* which destroy the following pesticides: chlorothiazide, juglone, semiquinone, nitrolon, kartocid and karate ("Evolution of soil microorganisms under the influence of pesticides". Proceedings of the Saratov State University, Scientif. Section "Chemistry and Biological Ecology", 2007, vol. 7, No. 2, p. 66).

Patent RU 2410170 describes a method for purification of contaminated soil from organic compounds, including pesticides, for example, dichlorobiphenyl (DHB), by adding a sorbent, which is of glauconite breed, pretreated at 200-300 °C and strain of bacteria of the genus *Rhodococcus.*

In all these methods of detoxification of toxic chemicals (toxic substances) for the decomposition of specific chemicals by certain kinds of microorganisms are offered. Mostly there is described the decomposition of pesticides in the aquatic media by microorganisms utilizing xenobiotics as a sole source of carbon and energy, or there is required the additional use of sorbents. A significant drawback of all these cultures is their narrow specificity for a particular substrate, and that they do not have preparative form and are not used in practice for the treatment of water, industrial wastewater and soil contaminated by hard-degradable chemicals such as pesticides.

Patent RU 2093478 describes the association of bacterial strains *Pseudomonas putida* VKM 1301, *Bacillus subtilis* VKM B-1742D and *Rhodococcus erythropolis* VKM Ac-1339D in the ratio of 1:1:1 for purification of water and soil from oil and petroleum products and polymer additives contained in the drilling mud and its use. However, the use of this association for the purification of water, soil and industrial waste water contaminated with persistent against degradation chemicals such as pesticides, have not previously been known.

There is known biological product "Biava" (patent RU 2248255), which improves soil fertility and stimulates the natural microflora of the medium. Biological product contains amylolytic, proteolytic and nitrogen-fixing microorganisms such as bacteria genus *Pseudomonas, Bacillus.* The disadvantage of this product is its complex composition, comprising more than 25 types of different organisms not adapted for the associative interaction, which is complicated for production and for maintenance of the strains. The use of a biological product "Biava" by its introduction into the soil is also described in the said patent. This product is not a stimulant of plant growth and has no ability to degradation of persistent chemicals such as pesticides or herbicides from a group of chlorophenoxy acetic acid: 2,4-dichlorofenoxyacetic acid (2,4-D), trichlorophenoxy acetic acid (2,4,5, -T), phenol, 2,4-dichlorophenol, CPAA and other chemical compounds that are described below. These compounds are among the most consistent and persistent in the environment. Intake of these chemicals by e.g. a human can cause serious diseases of organs, tissues, and nervous system. Their presence in food is considered inadmissible (see, e.g. Tinsley, I. Chemical Concepts in Pollutant Behavior. Transl. from English. M.: Mir, 1992, p. 281).

### Disclosure of Invention

The objective of the present invention is to provide a new biological product for treatment (detoxification) of soil, water and industrial wastewater contaminated with persistent chemicals such as pesticides, phenols, as well as to provide the method of use of the product. The product "PHENOX" of the present invention improves the purification of the environment from toxic substances, and as a consequence, improves the quality of consumer products grown in the treated soil. In addition the microbial degradation of toxic substances to non-hazardous compounds carried out by bacterial cells occurs in the soil. Biological product shows high efficiency in the treatment of industrial wastewater.

The present invention refers to the bioproduct ("PHENOX") for treatment of ground, soil, industrial wastewater from chemicals resistant to degradation such as pesticides. The offered bioproduct is an association of new strains of bacteria *Pseudomonas putida, Bacillus subtilis* and *Rhodococcus erythropolis* in a mass ratio of (1-2):(1-2):(1), which may further comprise a sorbent or to be immobilized on a sorbent, and mineral, organic or stimulating additives.

The new strain *Pseudomonas putida* was deposited in the Russian National Collection of Industrial Microorganisms (VKPM) under the accession number B-10997 (SEQ ID No. 3).

The new strain *Bacillus subtilis* was deposited in the Russian National Collection of Industrial Microorganisms (VKPM) under the accession number B-10999 (SEQ ID No. 1).

The new strain *Rhodococcus erythropolis* was deposited in the Russian National Collection of Industrial Microorganisms (VKPM) under the accession number Ac-1882 (SEQ ID No. 2).

The difference of the proposed product "PHENOX" compared with the prior art solutions is the specified association of strains of bacteria and the given preferable mass ratio.

### Brief Description of Drawings

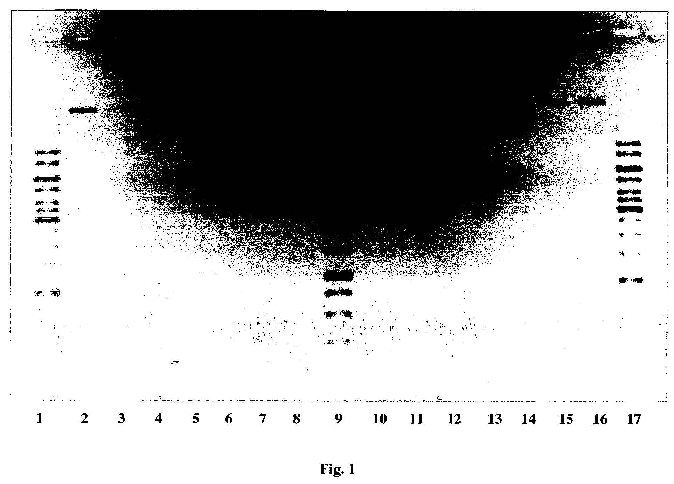

Fig. 1 is a plasmid profile of strains: 2 - 9 *Pseudomonas putida* VKPM B-10997, 10 - 17 *Rhodococcus erhytropolis* VKPM Ac-1882

The product is particularly effective when used for the decomposition of persistent pesticides, selected from the group of chlorofenoxyacetic acids such as 2,4-dichlorofenoxyacetic acid (2,4-D), trichlorofenoxyacetic acid (2,4,5-T), 2,4-dichlorophenoxy-α-propionic acid (Dichloroprop, 2,4-DR), 2-methyl-4-chlorophenoxy-α-propionic acid (Mekoprop, 2M-4HP, MSRR), 2,4,5-trichlorophenoxy-α-propionic acid (2,4,5-TP, Silvex), 2,4-dichlorophenoxy-α-oil (2,4-DV), methyl-[1-[(butylamino) carbonyl]-1H-benzimidazole-2-yl]carbamate and the product on its basis (benomyl, carbendazin, benazol); imidor, wherein the active substance is neonicotinoid imidacloprid; zontran, wherein the active substance is metribuzin, HCCH (hexachlorocyclohexane, hexachloran hexatox, dolmix, sineks) CPAA (chlorofenoxyacetic acid), PAA (fenoxyacetic acid), hexachlorophenol, 1,1-di(4'-chlorophenyl) -2,2-dichloroethane (DDT) and 2,4-dichlorophenol and phenols. Particularly the product is effective when used for the decomposition of persistent pesticides from the group of chlorofenoxyacetic acids, such as - 2,4-dichlorofenoxyacetic acid (2,4-D), trichlorofenoxyacetic acid (2,4,5-T), chlorofenoxyacetic acid (CPAA), fenoxyacetic acid (PAA), as well as 2,4-dichlorophenol and phenol, and imidacloprid.

The product of the present invention unlike to the previously known products has additional growth stimulating effect on seed germination and growth of cultivated plants, and also has fungicidal activity.

The invention comprises also a method for use of the offered biological product "PHENOX". Method consists of introducing of an effective amount of the product in contaminated ground, soil or wastewater.

Preferably, the product is introduced in an amount of 10 kg of dry matter per 1 ha of land during ploughing at an ambient temperature, preferably at a temperature of 15°-35° C. The preferred methods of administration are in the form of an aqueous solution by irrigation. The product can also be introduced to the ground by seeds treated with the product suspension with addition of biogenetic elements, such as vermicompost, or the introduction of a dry formulation of the product in the form of powder into the ground during ploughing or sowing.

The product can be introduced into the soil also by sowing seeds treated with the product suspension with the addition of additives, such as vermicompost. The product is diluted with water in the container by addition of vermicompost, nitrogen and phosphorus fertilizers.

The introduction of a dry formulation of the product in the form of powder in the ground during of ploughing or seeding is possible. The product "PHENOX" proposed according to the invention has a stimulating effect on plant growth, and strains of the product - good ability for adaptation to soil, which leads to an efficient detoxification of the environment. Strains of *Pseudomonas putida* and *Rhodococcus erythropolis* carry D-plasmids of the degradation and are able to transmit the property of destruction of pesticides and other harmful substances to indigenous bacterial populations in soil, water, wastewater, thereby increasing clearing from pollutants. The advantage of using this product is the decomposition of pesticides in soil for up to 86% and in liquid medium up to 99%.

The active basis of the offered product is the association of soil bacterial strains *Pseudomonas putida, Bacillus subtilis, Rhodococcus erythropolis.* All these cultures have polisubstratum specificity simultaneously to multiple xenobiotics: chlorofenoxyacetic acids, such as - 2,4-dichlorofenoxyacetic acid (2,4-D), trichlorofenoxyacetic acid (2,4,5-T), phenol, 2,4-dichlorophenol, CPAA and other specified in the examples. Their mixture ensures enhanced purification function of the environment from toxic compounds. As sorbents kaolin and finely crushed peat are used also serving as an organic additive; as organo-mineral and growth stimulating supplements vermicompost, its extract and chitin containing substrates. Before use dry powder of the product is mixed well in the container (bucket, jar) with warm water (25-30 °C) until a homogeneous suspension. 10 g of the product is stirred into 10 liters of water. It is recommended to add azophoska - ammonium nitrate based compound fertilizer (5 g/10 1) or compost (5 g/10 1), suspension periodically is stirred for aeration and microbial activation within 1-3 hours before use (watering the soil by watering-can (10-201/10 m²) or treatment of seeds 100-150 ml/100 g).

The biological product is obtained by a separate submerged cultivation, usually within 20-30 hours 3 strains of the said bacteria on the standard biotechnology equipment. Seeds are being obtained by growing strains in synthetic medium with the addition of pesticides and this material is being used for seeding large volume of fermenters.

Accumilated dry biomass of cells of the strains *Pseudomonas putida, Bacillus subtilis, Rhodococcus erythropolis* is mixed in a ratio of (1-2):(1-2):1. The total titre presents at least 5x10⁸ CFU/g of dry product after addition of carrier-sorbent, and other additives (the source concentrate of 10¹⁰⁻¹¹ and at least 5x10⁸ CFU/mL liquid product). For the industrial production and packaging of the proposed product the standard biotechnological and packaging equipment and materials are used.

The offered bioproduct ("PHENOX") eliminates penetration into plants, vegetables and berries plant protection chemicals, stimulates seed germination and plant growth, inhibits growth of fungal phytopathogens.

"PHENOX" is used for treating of contaminated by chemicals such as pesticides, phenol, possibly oil - water, industrial wastewater, soil and plant seeds. The strains contained in "PHENOX" are new.

The strain *Bacillus subtilis* B-10999 is isolated by the enrichment culture method from soil samples from the industrial zone of enterprises of chemical industry of the South Ural. The strain *Bacillus subtilis* was deposited in the Russian National Collection of Industrial Microorganisms (VKPM) under the number B-10999. The strain of *Bacillus subtilis* is identified on the basis of nucleotide sequence analysis of 16S rRNA gene (SEQ. ID No. 1). The nucleotide sequence (length not less than 500 base pairs) of DNA fragment encoding the 16S rRNA gene, has similarity with the declared type of 99%.

Culture-morphological features. The strain *Bacillus subtilis* has typical culture-morphological features for this kind. Rods, the size of which is 0.2-0.5 at 1.5-3 µm, single, often in pairs. They form oval and round endospores. The flagella are placed over the entire cell's surface. When grown on meat-peptone broth the culture forms developed matte film, the media, however, remains clear. When shaking in liquid medium, the complete dispersion of biomass does not occur. Colonies are dry, finely wrinkled, with wavy edge. On slices of potato growing is abundant in the form of colonies of a cream colour smooth, strongly big size plicate. Gram-positive.

Physiological and biochemical features. The strain *Bacillus subtilis* grows well on glucose-peptone medium, LB agar and broth, glucose-mineral medium at a temperature of 28 °C. It grows in media containing L-proline, DL-leucine, α-ketoglutarate, DL-α-alanine, L-glutamine, D(+)-xylose, L-asparagine, chitin, DL-serine, glucose, phenol and chlorophenoxy carbolic acids as the sole source of carbon. The strain *Bacillus subtilis* can be stored in a lyophilized state. Checking of the viability of the strain is carried out by planting of it on glucose-peptone agar, M9 and LB - once in 12 months. When it is stored on slants of these media at 5 °C - replanting is carried out every 3 months. The strain *Bacillus subtilis* is a non-pathogenic and non-toxic micro-organism. Intravenously infected white mice do not show zoo pathogenic properties.

The strain *Rhodococcus erythropolis* Ac-1882 is isolated by the enrichment culture method from soil samples from the industrial zone of enterprises of chemical industry of the South Ural. The strain *Rhodococcus erythropolis* was deposited in the Russian National Collection of Industrial Microorganisms (VKPM) under the number Ac-1882. The strain *Rhodococcus erythropolis* is identified on the basis of nucleotide sequence analysis of 16S rRNA gene (SEQ. ID No. 2). The nucleotide sequence (length not less than 500 base pairs) of DNA fragment encoding the 16S rRNA gene, has similarity with the declared type of 99%.

Culture-morphological features. The strain *Rhodococcus erythropolis* has typical culture-morphological features for this kind. Cells rod-shaped, rarely branched, the diameter 0.6-0.8 µm, the length - 3-8 µm. Cells were moderately polymorphic, often arranged in a V-shape, have an explicit cycle of development coccus-rod-coccus: at the age of 24-36 hours rod-shaped cells begin to shorten and the share of coccoid and oval cells increase. Grown-up colonies are pigmented, orange coloured. The consistency of colonies is paste-like. Gram-positive.

Physiological and biochemical features. The strain *Rhodococcus erythropolis* grows well on glucose-peptone medium, LB agar and broth, glucose-mineral medium at a temperature of 28 °C. It grows in media containing L-proline, D-arabinose, DL-α-alanine, L-glutamine, D(+)-xylose, L-asparagine, DL-serine, casein, glucose, phenol and chlorophenoxy carbolic acids as the sole source of carbon. The strain *Rhodococcus erythropolis* may be stored in a lyophilized state. Checking of the viability of the strain is carried out by planting of it on glucose-peptone agar, M9 and LB - once in 12 months. When it is stored on slants of these media at 5 °C - replanting is carried every 3 months. The strain *Rhodococcus erythropolis* is a non-pathogenic and non-toxic micro-organism. Intravenously infected white mice do not show zoo pathogenic properties.

The strain *Pseudomonas putida* B-10997 is isolated by the enrichment culture method from soil samples from the industrial zone of enterprises of chemical industry of the South Ural. The strain *Pseudomonas putida* was deposited in the Russian National Collection of Industrial Microorganisms (VKPM) under the number B-10997. The strain *Pseudomonas putida* is identified on the basis of nucleotide sequence analysis of 16S rRNA gene (SEQ. ID No. 3). The nucleotide sequence (length not less than 500 base pairs) of DNA fragment encoding the 16S rRNA gene, has similarity with the declared type of 99%.

Culture-morphological features. The strain *Pseudomonas putida* has typical culture-morphological features for this kind. Motile rods with polar located flagella. The size of the cells is 1-4 on 1.3-5.4 µm. Colonies on meat-peptone agar are grayish coloured, within the colonies and at their reverse side there is a reddish-brownish pigment. Spores do not occur. The consistency of the colonies is viscid. Gram-negative.

Physiological and biochemical features. The strain *Pseudomonas putida* grows well on glucose-peptone medium, LB agar and broth, glucose-mineral medium at a temperature of 28 °C. Grows in media containing DL-tyrosine, DL-tryptophan, D(+)-xylose, D(-)-mannitol, L-glutamic acid, DL-leucine, α-ketoglutarate, DL-α-alanine,D-ribose, L(+)-arabinose, L-glutamine, cellulose, L-asparagine, chitin, DL-serine, pectin, glucose, casein, and phenol and chlorophenoxy carbolic acids as sole source of carbon. The strain *Pseudomonas putida* can be stored in a lyophilized state. Checking of the viability of the strain is carried out by planting of it on glucose-peptone agar, M9 and LB - once in 12 months. When it is stored on slants of these media at 5 °C - replanting is carried out every 3 months. The strain *Pseudomonas putida* is a non-pathogenic and non-toxic micro-organism. Intravenously infected rabbits do not show zoo pathogenic properties.

### Examples of implementation of the invention

### Example 1

Identification of plasmid degradation of pesticides in the strains. Destructive properties of bacteria can be determined by extrachromosomal elements [Don, Pemberton, 1985; Ghosaletal., 1985]. These elements - plasmids were found in the strains *Pseudomonas putida* VKPM B-10997 and *Rhodococcus erhytropolis* VKPM Ac-1882. They were isolated by alkaline lysis method (Maniatis T., Fritsch, E., and Sambrook, J. Methods of genetic engineering. Molecular cloning: Trans. from English. / Ed. AA Baev. - M.: Mir, 1984. p. 480) from the cells of these strains and then plasmid products were fractionated in agarose gel under standard conditions (Maniatis et al., 1984) - (see Fig. 1 Plasmid profile of strains: 2 - 9 *Pseudomonas putida* VKPM B-10997, 10 -17 *Rhodococcus erhytropolis* VKPM Ac-1882).

### Example 2

Compatibility of strains contained in the product. The absence of an antagonistic activity between strains of the bacteria *Bacillus subtilis* VKPM B-10999, *Rhodococcus erythropolis* VKPM Ac-1882 and *Pseudomonas putida* VKPM B-10997 of the product was determined by the growth of strains in the contact lines in the media and by the method of agar blocks. On the LB agar medium the stroke of strain *B. subtilis* VKPM B-10999 was applied and perpendicular to it strokes of strains *Rhodococcus erythropolis* VKPM Ac-1882 and *Pseudomonas putida* VKPM B-10997 were applied. On the next cup on nutrient medium the stroke *Rhodococcus erythropolis* Ac-1882 was applied and perpendicular to it strains *Bacillus subtilis* B-10999 and *Pseudomonas putida* B-10997 were applied. On the third cup on medium the stroke of *Pseudomonas putida* B-10997 was applied and perpendicular to it the strains *Bacillus subtilis* B-10999 and *Rhodococcus erythropolis* Ac-1882 were applied. In the regions of contact lines inhibiting the growth of any of the strains was not observed. When the experiment was carried out using agar blocks, on the lawn of one of the strains on LB medium blocks of 3-days cultures of the other two strains were laid. No experiments showed inhibiting the growth of strains. This indicated the absence of antagonistic interactions between the strains and their possible effective joint application.

### Examples of compositions of the biological product "PHENOX"

### Example 3

Bioproduct "PHENOX", its form - a dry powder. Dry biomass of cells (concentrate) - *Bacillus subtilis* VKPM B-10999 no less than 5x10⁹ CFU/g, *Pseudomonas putida* VKPM B-10997 at least 5x10⁹ CFU/g and *Rhodococcus erythropolis* VKPM Ac-1882 at least 5x10⁸ CFU/g in the ratio (1-2):1:(1-2) (part of mass - 76.5-90%); without or with the introduction of sorbent - finely milled peat - 1-10% and/or stimulating additives, also containing mineral salts and trace elements - vermicompost - 0.1-1%; water, not more than 10.0 %; the total titre of living cells for at least 10¹⁰ CFU/g.

### Example 4

Bioproduct "PHENOX", in the form of a dry powder. Dry biomass of cells - *Bacillus subtilis* VKPM B-10999, *Pseudomonas putida* VKPM B-10997 and *Rhodococcus erythropolis* Ac-1882 in the ratio 1:1:1 - 5-30%; sorbent - finely milled peat - 1-10% and/or stimulating supplement containing also mineral salts and trace elements - vermicompost - 0.1-1%; water, not more than 10.0%; sorbent and filler kaolin - up to 100%; the total titre of viable cells for at least 5×10⁸ CFU/g.

### Example 5

Bioproduct "PHENOX", in the form of a dry powder. Dry biomass of cells - *Bacillus subtilis* VKPM B-10999, *Rhodococcus erythropolis* Ac-1882 and *Pseudomonas putida* VKPM B-10997 in the ratio 1:1:1 in total - 5-30%; water, not more than 10.0%; kaolin - up to 100%; the total titre of viable cells for at least 5×10⁸ CFU/g.

### Example 6

Bioproduct "PHENOX" in liquod form: cells of bacteria *Bacillus subtilis* VKPM B-10999, *Rhodococcus erythropolis* Ac-1882 and *Pseudomonas putida* VKPM B-10997 in the ratio 1:1:1 (total titre - 10⁹ CFU/ml) in culture medium without additives or with addition of biologically active substances (extract of compost or vermicompost - 1-5 ml/l or finely milled compost or vermicompost 0.1% and/or chitin or chitosan or chitin containing raw materials - 0.05-0.4%). Possible to store at 0-8 °C for at least 3 months, at temperatures of 18-25 °C up to 15 days. When stored at temperatures of 18-25 °C it is allowable to decrease the number of viable cells to a titre of 10⁷ CFU/ml.

### The method of use of the offered product is explained by the following examples:

### Example 7

Use of the product "PHENOX" for treatment of water from imidacloprid. The product "PHENOX" in the form of a dry powder in the following composition: concentrate of the bacterial cells in ratio of 1:2:1 with a total titre 5x10¹⁰ CFU/g (the composition of the product according to Example 1) in the amount of 100 mg was introduced in 200 ml of tap water contaminated with a pesticide (insecticide) imidacloprid at concentrations of 1 mg/L and 2 mg/l of an active ingredient. In addition in the aquatic medium additives of mineral salts were added (g/l): Na₂HPO₄.7H₂O - 6.4; KH₂PO₄ - 1.5; NaCl 0.25 g/l; NH₄Cl 0.5 g/l. For the control the samples of water contaminated with imidacloprid at concentrations of 1 mg/L and 2 mg/l with mineral salts without biological product were used. The samples were incubated at ambient temperature with constant stirring in a shaker (120 rpm). The experiment was repeated 3 times. The content of imidacloprid in water was determined by standard chromatographic methods at the 1st, 3rd, 5th, 7th and 10^{th} day (Rudakov et al, 2004). The coefficient of variation of data was no higher than 5%.

**Table 1**

| Dynamics of the content of imidacloprid in water when using the product "PHENOX" | | | | | |
|---|---|---|---|---|---|
| The variation | The content of imidacloprid, mg/l | | | | |
| | 0 day | 1^{st} day | 3^{rd} day | 7^{th} day | 10^{th} day |
| The control - imidacloprid 1 µkg/ml | 1.00 | 0.98 | 0.98 | 0.94 | 0.96 |
| The control - imidacloprid 2 µkg/ml | 2.00 | 2.09 | 2.01 | 1.93 | 1.95 |
| Biological product + imidacloprid 1 µkg/ml | 1.00 | 0.94 | 0.81 | 0.48 | 0.28 |
| Biological product + imidacloprid 2 µkg/ml | 2.00 | 1.57 | 1.15 | 0.83 | 0.64 |

The use of a biological product leads to a significant reduction of the content of imidacloprid in contaminated water. Reducing of the concentration of pesticide at the starting contamination of water by imidacloprid 1 mg/l at the 10^{th} day was 0.28 mg/l, (72% less). At contamination of water at a dose of 2 mg/l introduction of the biological product reduces the concentration of imidacloprid to 0.64 mg/l, (68% less). These data indicate that the biological product "PHENOX" allows purification of water from imidacloprid.

### Example 8

The use of a biological product "PHENOX" for the purification of water from phenol. Biopproduct "PHENOX" in the liquid form of the following composition: bacterial cells at a ratio of 1:1:1 with a titre of 5×10⁹ CFU/g (a product composition according to Example 1, without addition of stimulating ingredients) in the amount of 0.01% of the volume was introduced into the water contaminated with phenol (the content of phenols is 100 mg/l). In the aquatic media additionally mineral nitrogen-phosphorus fertilizer in an amount of 0.5 g/l was introduced. For aeration and mixing air was being periodically supplied by a pump in the container with contaminated water. The temperature of water was maintained about 22-25 °C. The determination of phenol in the water was conducted by a standard photometric method (Rudakov et al, 2004). The experiment was repeated 3 times. The coefficient of variation of data was no higher than 7%.

**Table 2**

| Dynamics of the content of phenol after treatment of water with the product "PHENOX" | |
|---|---|
| Incubation time | The concentration of phenol, Mg/l |
| 0 day | 100.0 |
| 1^{st} day | 73.0 |
| 3^{rd} day | 67.0 |

When using the product "PHENOX" the concentration of phenol in the water during the first day decreases by 27%, and till the 3^{rd} day - by 33% (Table 2).

### Example 9

Use of the product 'PHENOX' for the purification of water from dichlorophenol. The experiment was carried out according to Example 8, but using the ratio of strains of 2:1:1 and water contaminated with dichlorophenol 2,4- DCP (the content of dichlorophenol was 100 mg/l).

**Table 3**

| Dynamics of the content of dichlorophenol after treatment of water with the product "PHENOX" | |
|---|---|
| Incubation time | The concentration of 2,4-DCP, mg/l |
| 0 day | 100.0 |
| 1^{st} day | 30.0 |
| 3^{rd} day | 21.9 |

When using the product "PHENOX" for the purification of water from dichlorophenol the concentration of 2,4-DCP during the 1st day of incubation decreases at 70% (Table 3).

### Example 10

Use of the product 'PHENOX' for the purification of water from 4-chlorophenoxy acetic acid. The experiment was carried out according to Example 8, but using the ratio of strains 1:2:1 and water contaminated with 4-4-chlorophenoxy acetic acid 4-CPAA (the content of 4-CPAA was 100 mg/l).

**Table 4**

| Dynamics of the content of 4-chlorophenoxy acetic acid after treatment of water with the product "PHENOX" | |
|---|---|
| Incubation time | The concentration of 4-CPAA, mg/l |
| 0 days | 100.0 |
| 1 day | 96.3 |
| 3 days | 95.8 |
| 5 days | 94.1 |
| 7 days | 92.5 |
| 9 days | 90.0 |
| 11 days | 68.7 |
| 13 days | 63.9 |
| 15 days | 62.0 |

The use of the product 'PHENOX' for the purification of water from 4-chlorophenoxy acetic acid by the 9^{th} day reduces the concentration of 4-CPAA in water for about 10%, and by the 15^{th} day - for 38.0% (Table 4).

### Example 11

Use of the product 'PHENOX' for the purification of water from 2,4-dichlorofenoxyacetic acid. The experiment was carried out according to Example 8, but using water contaminated with 2,4-dichlorofenoxyacetic acid 2,4-D (the content of 2,4-D was 100 mg/l).

**Table 5**

| Dynamics of the content of 2,4-dichlorofenoxyacetic acid after treatment of water with the product "PHENOX" | |
|---|---|
| Incubation time | The concentration of 2,4-D, mg/l |
| 0 days | 100.0 |
| 1 day | 95.3 |
| 3 days | 89.4 |
| 5 days | 81.5 |
| 7 days | 74.3 |
| 9 days | 70.0 |
| 11 days | 68.6 |
| 13 days | 66.9 |
| 15 days | 65.0 |

When using the product 'PHENOX' for the purification of water from 2,4-dichlorofenoxyacetic acid the content of 2,4-D till the 3rd day gradually falls for 11%, till the 9^{th} day for 30% and the 15^{th} day for 35%.

### Example 12

Use of the product 'PHENOX' for the purification of water from 2,4,5-trichlorofenoxyacetic acid. The experiment was carried out according to Example 8, but using water contaminated with 2,4,5-trichlorofenoxyacetic acid 2,4,5-T (the content of 2,4,5-T was 100 mg/l).

**Table 6**

| Dynamics of the content of 2,4,5-trichlorofenoxyacetic acid after treatment of water with the product "PHENOX" | |
|---|---|
| Incubation time | The concentration of 2,4,5-T, mg/l |
| 0 days | 100.0 |
| 1 day | 96.3 |
| 3 days | 85.0 |
| 5 days | 64.8 |
| 8 days | 55.0 |

When using the product 'PHENOX' for the purification of water from 2,4,5-trichlorofenoxyacetic acid the concentration of 2,4,5-T to the 3^{rd} day reduces for 15%, to the 8^{th} day for 45% compared with the initial level (Table 6).

### Example 13

Use of the product 'PHENOX' for the purification of water from phenol. The experiment was carried out according to Example 12, but using the product 'PHENOX' in the form of a dry powder (according to Example 3, with a titre of 5×10¹¹ CFU/g and the introduction of vermicompost - 0.1%).

**Table 7**

| Dynamics of the content of phenol after treatment of water with the product "PHENOX" | |
|---|---|
| Incubation time | The concentration of phenol, mg/l |
| 0 days | 100.0 |
| 1 day | 70.0 |
| 3 days | 45.0 |

When using the product 'PHENOX' in the form of a dry powder with catalytic additive for the purification of water from phenol its content reduces by the end of the 1^{st} day for 30%, by the end of the 3^{rd} day for 55% compared with the initial level (Table 7).

### Example 14

Use of the product 'PHENOX' for the purification of water from 2,4-Dichlorofenoxyacetic acid. Experiment was carried out according to Example 13, but using the product according to Example 3, without vermicompost with a titre of 10¹¹ CFU/g and water contaminated with 2,4-dichlorofenoxyacetic acid, 2,4-D (the content of 2,4-D was 100 mg/l).

**Table 8**

| Dynamics of the content of 2,4-dichlorofenoxyacetic acid after treatment of water with the product "PHENOX" | |
|---|---|
| Incubation time | The concentration of 2,4-D, mg/l |
| 0 days | 100.0 |
| 2 days | 81.3 |
| 4 days | 64.2 |
| 6 days | 61.8 |
| 8 days | 60.5 |
| 10 days | 59.0 |

When using the product 'PHENOX' for the purification of water from 2,4-dichlorofenoxyacetic acid leads to a gradual decrease of the concentration of 2,4-D - on the 10^{th} day it drops to 59% from the starting value (Table 8).

### Example 15

Use of the product 'PHENOX' for the purification of water from dichlorophenol. The experiment was carried out according to Example 13, but using water contaminated with dichlorophenol 2,4-DCP (the content of dichlorophenol was 100 mg/l).

**Table 9**

| Dynamics of the content of dichlorophenol in the treatment of water with the product "PHENOX" | |
|---|---|
| Incubation time | The concentration of 2,4-DCP, mg/l |
| 0 days | 100.0 |
| 2 days | 96.2 |
| 4 days | 91.4 |
| 6 days | 86.9 |
| 8 days | 80.5 |
| 10 days | 75.7 |
| 12 days | 70.1 |
| 14 days | 64.9 |
| 16 days | 60.8 |
| 18 days | 58.7 |
| 20 days | 56.6 |
| 22 days | 53.0 |

When using the product 'PHENOX' for the purification of water from dichlorophenol the concentration of 2,4-DCP in 22 days drops drops to 53% from the starting value (Table 9).

### Example 16

Use of the product 'PHENOX' for the purification of water from 2,4,5-trichlorofenoxyacetic acid. The experiment was carried out according to Example 8, but using the product with stimulant additives and titres 10⁹ CFU/g and water contaminated with 2,4,5-trichlorofenoxyacetic acid 2,4,5-T (the content of 2,4,5-T was 100 mg/l).

**Table 10**

| Dynamics of the content of 2,4,5-trichlorofenoxyacetic acid in the treatment of water with the product "PHENOX" | |
|---|---|
| Incubation time | The concentration of 2,4,5-T, mg/l |
| 0 days | 100.0 |
| 2 days | 70.0 |
| 4 days | 35.2 |
| 6 days | 33.8 |
| 8 days | 33.1 |
| 10 days | 32.6 |
| 12 days | 31.0 |
| 14 days | 31.9 |
| 16 days | 38.7 |
| 18 days | 35.8 |
| 20 days | 28.6 |
| 22 days | 30.1 |
| 24 days | 31.4 |
| 26 days | 18.3 |
| 28 days | 16.0 |

The content of 2,4,5-T in water consistently reduces after treatment by 'PHENOX' - till the 2^{nd} day for 30%, till the 12^{th} day for 69%, and till the 28^{th} day for 84% (Table 10).

### Example 17

Use of the bioproduct 'PHENOX' for the purification of wastewater from phenols. Bioproduct 'PHENOX' in the form of a dry powder of the following composition: dry biomass of cells - *Bacillus subtilis* VKPM B-10999, *Rhodococcus erythropolis* Ac-1882 and *Pseudomonas putida* VKPM B-10997 in a ratio 1:1:1 in the amount - 10%, vermicompost - 1%, water not more than 10%, kaolin - up to 100%, titre of alive cells - 2×10⁹ CFU/g (prepared according to Example 4). Before using a dry powder of the product was mixed well in the container (bucket, jar) in warm water (25-30 °C) until obtaining a homogeneous suspension. In the container having a working volume, equipped with a stirrer or a device for bubbling air, 1.0 kg of the biological product is introduced, 0.5 kg of azophoska and 200 l of warm technical water was added. To activate the microorganisms the suspension was stirred or perflated with air for 24 hours. Then a working suspension of the product was prepared and 10 kg of fertilizer (azophoska) and 1 kg of lime was added to the wastewater having volume of 10 m³ in the container. The container with wastewater was perflated by air daily. Wastewater pollution of "Ufahimprom" by phenolic compounds was 30.4 mg/liter. The efficiency of the treatment of wastewater from phenols by biological product 'PHENOX' was evaluated without additives and with addition of mineral fertilizers in the wastewater (Table 11).

**Table 11**

| Dynamics of the decontamination of wastewater from phenols using biological product "PHENOX" | | | | | | | | |
|---|---|---|---|---|---|---|---|---|
| Variation | Period after the introduction of strain of a biological product 'PHENOX' into the wastewater, days | | | | | | | |
| | The content of phenol, *mg*/*l* | | | | The efficiency of the treatment of wastewater from phenols, % | | | |
| | 0 | 2 | 5 | 7 | 0 | 2 | 5 | 7 |
| Wastewater | 30.4 | 10.2 | 0.01 | 0.01 | 0 | 66.400 | 99.970 | 99.970 |
| Wastewater with addition of mineral salts | 30.4 | 3.2 | 0.001 | 0.001 | 0 | 89.500 | 99.997 | 99.997 |

The degree of purification of wastewater from phenols on the second day after the introduction of the biological product 'PHENOX' reached 66.4%, and on the 5^{th} day was for more than 99%. Effectiveness of the use of the biological product 'PHENOX' for the treatment from phenols increases by the addition of mineral salts in the wastewater, already on the 2^{nd} day the degree of purification of wastewater from phenol reached 89.5%, and on the 7^{th} day - wastewater was released from phenols by more than 99.997% (Table 11).

### Example 18

Use of the biological product 'PHENOX' for the treatment of industrial wastewaters from phenols. Treatment of industrial wastewaters was carried out according to the previous example, without addition of fertilizer, but using wastewater of LLC "Novo-Ufimsky NPZ" and JSC "Dubitel".

**Table 12**

| Dynamics of the decontamination of industrial wastewater from phenols by using the biological product PHENOX | | | | | | | | |
|---|---|---|---|---|---|---|---|---|
| Variation | Period after the introduction of the biological product 'PHENOX' in the wastewater, in days | | | | | | | |
| | The content of phenol, *mg*/*l* | | | | The content of phenol, *mg*/*l* | | | |
| | 0 | 1 | 3 | 7 | 0 | 1 | 3 | 7 |
| Wastewater of a petrochemical company | 0.096 | 0.064 | 0.012 | 0.012 | 0 | 66.7 | 87.5 | 87.5 |
| Wastewater of tanning industry | 0.750 | - | 0.155 | 0.155 | 0 | - | 79.4 | 79.4 |

The degree of purification of wastewater from phenols of the petrochemical enterprise on the first day after the introduction of biological product 'PHENOX' was 66.7% and on the 3-7^{th} day reaches 87.5%. The efficiency of use of the biological product 'PHENOX' for the purification of wastewater of tanning industry from phenol during 3-7 days is 79.4% (Table 12).

### Example 19

Use of the product 'PHENOX' for the destruction of oil in an aqueous medium. The product in the form of dry powder (composition according to Example 3) with a titre of 1× 10¹⁰CFU/g in the amount of 100 mg was introduced in 200 ml of water, contaminated with crude oil (at a concentration of 1%) with addition of salts (K₂HPO₄ - 0.05%, NH₄Cl - 0.05%, CaCO₃ - 0.001%). The oil-contaminated water after inoculation of product and mineral salts of nitrogen, phosphorus, potassium and lime was incubated for 10 days at ambient temperature under static conditions with daily stirring. Control was made by variation without introduction of the product. The experiment was repeted for 3 times. On the third day dark flakes in the volume of water and smaller than in the control variation stains of oil on the surface were observed. On the 5^{th} day in the variation with the product a small amount of oil stains on the surface of water was observed, about for a half the film of oil was destroyed on the walls of the flask. By the 10^{th} day of the experiment a small amount of oil on the surface of water and on the walls of the container was observed. Mass concentration of oil in the samples was measured on the 10^{th} day in the concentratometer KH-2 (manufacturing company LLC PEP SIBEKOPRIBOR, Novosibirsk) by the spectrophotometric method in terms of selective absorption of petroleum products in the infrared area of spectrum at a wavelength of 3.42 micrometers [The method of measurement of mass concentration of NP in the samples of drinking water, natural and wastewater by infrared spectrophotometry PND F 14.1:2:4.168-2000. Novosibirsk, 1998, 17 p.]. The coefficient of the variation of data - no more than 5%. Use of the product reduces the amount of oil in water for 28% (from 2652 mg/l in control variation up to 1915 mg/ml in the variation with the biological product).

### Example 20

Adatation of bacterial strains of the product in the soil. Dynamics of the population of bacterial strains of the product in the soil was studied in model experiments. A suspension of cells in the amount of 3×10⁸ cells per 1 g of soil was placed in glass flask with 50 g of leached top soil. The experiment included 3 variations: without xenobiotic (the control) and 2,4-D at concentrations of 100 and 10 000 MPC. MPC for the 2,4-D in soils - 0.1 mg/kg. Soil moisture was 60% of full capacity. Periodically the strains of the product 'PHENOX' were isolated on minimal salt agar medium M9 with the addition of 2,4-D as the sole source of carbon and energy. The number of colony forming units (CFU) of bacterial strains in the control soil increased within first 30 days, and then there was a decline of population density of these bacteria (Table 13). In polluted 2,4-D the density of the bacterial population was higher in the first 2.5 weeks after the introduction than in the controls, and then it gradually decreased. In the most polluted soil bacterial population of 'PHENOX' sharply increases and 10-50 times exceedes the population density of these strains in the control. This rise of the number of bacteria 'PHENOX' is due to using xenobiotic as a source of nutrition. Reducing the number of bacteria 'PHENOX' in the soil in this variation occurs after 30 days. By day 52^{nd} the density of the population of introduced strains in all variants was at a similar level, indicating a decrease of the concentration of 2,4-D in contaminated soils, as well as possible involvement in the degradation of pesticides of other microorganisms.

A good adaptation of the microorganisms to soils was revealed. Similar data were obtained in experiments with other types of soils - gray forest, podsol soil and xenobiotics in three concentrations of 100 MPC, 1000 MPC, 10 000 MPC. MPC for the 2,4-D in soils is 0.1 mg/kg. This shows the possibility of the use of the product of the present invention in these types of soils.

**Table 13**

| Dynamics of the number of strains of the product "PHENOX" introduced to the contaminated by pesticide 2,4-D leached top soil | | | | |
|---|---|---|---|---|
| Time | Dynamics of the number of cell population CFU x10⁵/g of soil | | | |
| | Net soil | Soil with 2,4-D 100 MPC | Soil with 2,4-D 1000 MPC | Soil with 2,4-D 10000 MPC |
| 0 days | 0.00 | 0.00 | 0.00 | 0.00 |
| 9 days | 0.37 | 4.33 | 4.33 | 0.66 |
| 19 days | 13.40 | 50.50 | 4.30 | 700.00 |
| 30 days | 70.00 | 55.40 | 9.00 | 340.50 |
| 52 days | 1.70 | 3.00 | 2.70 | 0.98 |

### Example 21

Use of a biological product "PHENOX" for the treatment of soil contaminated with pesticide 2,4,5-T (trichlorofenoxyacetic acid). Soil (100 g) of arable horizon was sieved through a sieve of 2 mm diameter and placed in glass flasks for 500 ml. 2,4,5-T was introduced in soil in the amount of 100 mg/g and mixed thoroughly. Bioproduct 'PHENOX' with a titre of 10⁹ CFU/g (according to Example 5) was inoculated into a bottle with the soil, moistened to 60% of full capacity and also mixed. Flasks were closed with cotton plugs with parafilm and incubated at constant humidity and temperature of 25 °C in a thermostat. Determination of 2,4,5-T in soil was carried out by the standard method (The method of pesticides measurement in soil, M.E., Medicine, 1984, - p. 256 p.). The experiment was repeated 3 times. The coefficient of variation of data was no higher than 8%.

**Table 13***

| Dynamics of the decontamination of soil from 2,4,5-T using the biological product "PHENOX" | | | | | | | |
|---|---|---|---|---|---|---|---|
| Parameter | Time after treatment, days | | | | | | |
| | 1 | 5 | 10 | 14 | 21 | 30 | 48 |
| The content of 2,4,5-T in soil, *mg*/g | 100 | 95.0 | 87.6 | 85.0 | 72.6 | 68.1 | 33.5 |
| The degree of purification comparing to control,% | 0 | 5.0 | 12.4 | 15.0 | 27.4 | 31.9 | 66.5 |

The degree of soil decontamination from 2,4,5-T by using the biological product 'PHENOX' in 14 days was 15%, in 21 days - 27.4%, and in 30 days - 31.9% and reached 66.5% by the 48^{th} day. The rate of decomposition of 2,4,5-T increases significantly after 30 days by incubation of soil with biological product 'PHENOX' (Table 13*).

### Example 22

Use of the product 'PHENOX' for the disintegration of pesticide (fungicide) TMTD in soil. The venue of the experiment: concrete ground, adjacent to the warehouse for storage of pesticides (The region of Altai, Biysk city, 4 Prigorodnaya Street). In summer time on an open concrete ground a suit plot area of 3 m² (1×3 m) was arranged, delimited by wooden partitions with height of 20 cm at 3 sites 1×1 m. The bottom of the plots was spread with plastic film to prevent the spread of pollutants from the soil to concrete ground. Plots were filled with a layer of top soil with thickness of 15 cm. At a height of 60 cm from the ground plots were covered by non-woven covering material (spunbond). In the soil the pesticide (fungicide) tetramethylthiuram disulfide (TMTD) at the rate of 50 g/m² was introduced, vermicompost at the amount 50 g/m², mineral nitrogen and phosphorus fertilizer enriched with micronutrients - 100 g/m² and organic fertilizers based on peat - 400 g/m² and mixed thoroughly. Product "PHENOX" in the form of dry powder composition: bacterial cells - 10%, vermicompost - 1%, kaolin - 89% with a titre of 5×10⁹ CFU/g (according to example 4) of 300 g was dissolved in 15 liters of water (25-30 °C) and incubated 24 hours with periodic agitation to improve aeration and activate microorganisms. The suspension of biological product was applied to a surface of the plot at the rate 5 litres/m² by sprinkling with a watering can. The surface of the plot was further watered by 5 litres/m² of soil. Soil contaminated with TMTD, after treatment with the product "PHENOX" was loosened to improve aeration and mulched by a thin layer of sawdust. If necessary, and in case of no rain the soil in the plot was occasionally loosened and moistened up to 60-70% of full capacity. To evaluate the effectiveness of soil decontamination from TMTD by the method of sample middling, samples were taken each 1.5 and 3 months. The quantitative content of TMTD in the samples was determined chromatographically. The coefficient of variation of data was not higher than 5%. 1.5 months after the treatment with the product "PHENOX" the content of TMTD in soil reduced by 53%, in 3 months - by 75%.

### Example 23

Use of the product 'PHENOX' for the decomposition of the pesticide (herbicide) dichloralurea in soil. The experiment was conducted similar to the Example 22. The pesticide (herbicide) dihloralurea was introduced into the soil at the rate of 50 g/m² 1.5 months after the treatment with the product "PHENOX" the content of tetramethylthiuram disulfide TMTD in soil reduced by 59%, in 3 months - by 78%.

### Example 24

Use of the product 'PHENOX' for the purification the soil from chlorinated aromatic compounds. Venue of the experiment: Rostovskaya Oblast, Salski region. Characteristics of the plot: land for agricultural purposes, contaminated with chlorinated aromatic compounds. In summer time in an open air a plot area of 530 m² (size of 66 m × 8 m) was marked, delimited in areas of 170 m² by protective zones of 0.5 m. The experiment was repeated 3 times. In contaminated soil vermicompost in the amount of 100 g/m², mineral nitrogen and phosphorus fertilizer at the rate of 10 g/m² were introduced. The product 'PHENOX' in the form of dry powder composition: bacterial cells - 10%, vermicompost - 1%, kaolin - 89% with a titre of 5×10⁹ CFU/g (according to Example 4) in an amount of 10 g/m² to obtain a working solution was dissolved in a container and mixed well in warm water (25-30 °C) until a homogeneous suspension. Working suspension of the product "PHENOX" was applied to the surface of the soil at the rate of 10 l/m². The application was carried out by sprinkling with any provided for this purpose machines and devices. The treatment was performed at an average daily soil temperatures at least +5 °C and not above +30 °C. The introduced biologic product and fertilizers were worked in the upper layer of the contaminated soil by plowing, digging, cultivation or other methods of tillage. The soil periodically was loosened to improve aeration and humidified up to 60-70% of full capacity (if necessary, in case of the absence of rain). After two weeks the soil at the site was plowed and humidified. To evaluate the effectiveness of soil decontamination from chlorinated aromatic compounds by the method of sample middling, samples were taken every 1.5 and 3 months. The quantitative content of aromatic compounds in the samples was determined by the standard method (Rudakov et al, 2004). The coefficient of variation of data was not higher than 9%. 1.5 months after the treatment with the product 'PHENOX' the content of aromatic compounds in soil reduced by 35%, in 3 months - by 68%.

### Example 25

Assessment of fungicidal activity of the product (in the ratio 1:1:1). The fungicidal activity of bacterial strains *B. subtilis* VKPM B-10999, *Rhodococcus erythropolis* VKPM Ac-1882 and *Pseudomonas putida* VKPM B-10997 of the product was determined by the standard method of agar blocks (Egorov, 1976). Petri dish with a solid nutrient medium was sown with phytopathogenic fungus lawn culture. Strains of fungi was used, that caused destruction of the roots, wilting, mottling, root rot - *Fusarium oxysporum, Alternaria tennuisima*, *Alternaria alternata,* and gray mold on leaves and fruits of plants - *Botrytis cinerea,* rotting of agricultural products - *Geotrichum candidum.* Each culture was sown into 2 cups of Czapek's medium and on its surface the blocks of bacterial strains were placed, cut out of 5-days lawn of bacteria on standard LB-agar. 4 blocks of the bacterial strain sown by phytopathogenic fungus on each medium were placed. Cups were incubated for 2 days at 5 °C (for the diffusion of antibiotic substances in the agar), and then - at 25 °C and the existence of zones of growth of the inhibition of fungi was established.

**Table 14**

| Fungicidal activity of bacterial strains of the product | | | | | |
|---|---|---|---|---|---|
| The bacterial strain | The size of areas of inhibition of the fungus on 3^{rd} day, mm | | | | |
| | *Fusarium oxysporum* | *Alternaria alternata* | *Alternaria tennuisima* | *Botrytis cinerea* | *Geotrichum candidum* |
| *Bacillus subtilis* | 1 | 3 | -* | 1 | |
| *Rhodococcus erythropolis* | 1 | 2 | - | 2 | 2 |
| *Pseudomonas putida* | 1 | 2 | 2 | 3 | 2 |

| | | | | | |
|---|---|---|---|---|---|
| * - There is no inhibition area | | | | | |

Fungicidal activity against all tested in the experiment of phytopathogenic fungi is established for the strain *Pseudomonas putida* B-10997, and for the strains of *B. sublilis* B-10999, *Rhodococcus erythropolis* Ac-1882 - to fungus causing predominantly root infections and decay (Table 14). That is the bacterial product has fungicidal activity to pathogens that determines various fungal diseases of plants.

### The plant growth stimulating activity of the product

### Example 26

Use of the product to stimulate plant growth of radish. The product was in the form of dry powder of the following composition: bacterial cells - 10% (dry biomass of cells *Bacillus subtilis* VKPM B-10999, *Pseudomonas putida* VKPM B-10997 and *Rhodococcus erythropolis* Ac-1882 in the ratio 1:1:1), , kaolin - 89% with a titre of 5×10⁹ CFU/g was dissolved in 10 ml of distilled water (dilution 1:10) and then sequential dilutions of 1:100 and 1:1000 were prepared. Radish seeds of the variety of 'Rose-red' with a white tip were spread on filter paper in Petri dishes for 25 seeds in each Petri dish. The experiment was repeated 4 times. Seeds were treated with the product suspension in an amount of 2 ml for a Petri dish by using an automatic dispenser. Control variations were the seeds germinated in distilled water and seeds treated with 0.05% solution of indole-butyric acid (IBA) - the stimulatator of the growth of plants of auxin type. Petri dishes with treated seeds were incubated under natural light and at ambient temperature in a humid chamber. The length of roots, the length of shoots and the dry weight of plants was determined at the 5^{th} day. The coefficient of variation of data was no higher than 5%.

**Table 15**

| Growth of radish plants when using the biological product "PHENOX" | | | |
|---|---|---|---|
| The variation of treatment | Parameter | | |
| | Length of the root, mm | Length of the seedling, mm | Dry weight (air-dry weight) g |
| The control (water) | 69 | 30 | 0.27 |
| The control (IBA) | 70 | 37 | 0.35 |
| The product dilution 1:10 | 61 | 41 | 0.34 |
| The product dilution 1:100 | 81 | 42 | 0.37 |
| The product dilution 1:1000 | 58 | 38 | 0.32 |

Suspension of the product stimulates the growth of the red radish and accumulation of biomass. The maximum effect was observed from the aqueous suspension of the product of 1:100. The product at a dilution of 1:100 increased the length of the 5 days radish roots from 69 to 81 mm (17%), length of seedling from 30 to 42 mm (40%) and dry weight of plants by 37%, from 0.27 to 0.37 g (Table 16).

### Example 26

Use of the product 'PHENOX' to stimulate plant growth of cabbage. The experiment was conducted similarly to the Example 25 (the content of the product: dry biomass of cells - *Bacillus subtilis* VKPM B-10999, *Pseudomonas putida* VKPM B-10997 and *Rhodococcus erythropolis* Ac-1882 in the ratio 1:1:1 - 10%; sorbent - finely milled peat - 89%, vermicompost - 1%). Seeds of cabbage variety 'Slava' were treated by the dilutions of the product. The length of roots and shoots of plants were determined after 5 days.

**Table 16**

| Growth parameters of cabbage variety 'Slava' when using the product | | |
|---|---|---|
| The variation of treatment | Parameter | |
| | Length of the root, mm | Length of the seedling, mm |
| The control (water) | 46 | 31 |
| The control (IBA) | 52 | 36 |
| The product dilution 1:10 | 64 | 35 |
| The product dilution 1:100 | 69 | 37 |
| The product dilution 1:1000 | 63 | 37 |

Aqueous suspension of the product (at all tested dilutions) increases the length of roots up to 50% - from 46 to 63-69 mm and length of shoots by 19% - from 31 to 37 mm at a dilution of 1:100 and 1:1000 (Table 16). The results indicate a strong stimulating effect of the product on the growth of roots of cabbage variety 'Slava'.

### Example 27

Use of the product to stimulate the clover seed germination and plant growth. The experiment of conducted as in the Example 26. The red clover seeds were treated with the dilutions of the product. After 5 days the length of the root and the length of the seedling, germination and dry weight of plants were determined.

**Table 17**

| Growth parameters of the red clover when using the biological product "PHENOX" | | | | |
|---|---|---|---|---|
| The variation of treatment | Parameter | | | |
| | Length of the root, mm | Length of the seedling, mm | Germination, % | Dry weight, g |
| The control (water) | 18 | 30 | 88 | 0.07 |
| The control (IBA) | 23 | 33 | 91 | 0.09 |
| The product dilution 1:10 | 34 | 40 | 84 | 0.12 |
| The product dilution 1:100 | 24 | 33 | 100 | 0.15 |
| The product dilution 1:1000 | 25 | 36 | 96 | 0.12 |

The product at a dilution of 1:10 increases the linear dimensions of the plant - root length by 89% (from 18 to 34 mm) and length of the seedling by 33% (from 30 to 40 mm). The product at a dilution of 1:100 enables improvement of germination by 12% - from 88 to 100% and the increase of dry weight by 114% - from 0.07 to 0.15 g (Table 17). Thus, the product has a strong stimulatory effect on root growth and increase of plant mass of clover.

### Example 27

Use of the product to stimulate the cucumber plant growth. Experiment was conducted as in the Example 26. The seeds of cucumber variety 'Russkaya Rubashka F1' were treated by the dilutions of the product. The length of root and seedling, germination and plant dry weight was determined after 6 days.

**Table 18**

| Growth parameters of cucumber plants when using the biological product "PHENOX" | | | | |
|---|---|---|---|---|
| The variation of treatment | Parameter | | | |
| | Length of the root, mm | Length of the seedling, mm | Germination, % | Dry weight, g |
| The control (water) | 46 | 33 | 84 | 0.46 |
| The control (IBA) | 59 | 38 | 92 | 0.55 |
| The product dilution 1:10 | 74 | 41 | 96 | 0.72 |
| The product dilution 1:100 | 71 | 41 | 100 | 0.70 |
| The product dilution 1:1000 | 64 | 36 | 92 | 0.63 |

Aqueous suspensions of the product have a stimulating effect on the growth of cucumber variety Russkaya Rubashka F1. For example, in an aqueous dilution of 1:10 product increases the length of the root at 61% - from 46 to 74 mm, the length of the seedling at 24% - from 33 to 41 mm, the germination at 16% - from 84 to 100% and dry weight of plants by 37 % - from 0.46 to 0.63 g (Table 18). The greatest effect of the product is on root growth of cucumber.

### Example 27

Use of the product to stimulate the beet seed germination. The experiment was conducted similarly to the Example 26. Beet seed variety 'Detroit' was treated by the dilutions of the product. Seed germination was determined on the 7^{th} day.

**Table 19**

| Germination parameters of beet seed when using the biological product "PHENOX" | |
|---|---|
| The variation of treatment | Parameter |
| | Germination,% |
| The control (water) | 92 |
| The control (IBA) | 95 |
| The product dilution 1:10 | 96 |
| The product dilution 1:100 | 99 |
| The product dilution 1:1000 | 96 |

The product at a dilution of 1:100 increases germination of beet seeds by 7% - from 92 to 99% (Table 19).

### Example 28

Use of the product to stimulate the growth of onions plants. The experiment was conducted similarly to the Example 27. Onion seeds of variety 'Karmen' were treated with the preparation at dilutions of 1:100 and 1:1000. The length of root and seedling, germination and plant dry weight is determined after 10 days.

**Table 20**

| Growth parameters of onion plants when using the biological product "PHENOX" | | | |
|---|---|---|---|
| The variation of treatment | Parameter | | |
| | Length of the root, mm | Length of the seedling, mm | Dry weight, g |
| The control (water) | 30 | 57 | 0.19 |
| The control (IBA) | 33 | 61 | 0.21 |
| The product dilution 1:100 | 34 | 69 | 0.28 |
| The product dilution 1:1000 | 37 | 62 | 0.25 |

The product at a dilution of 1:1000 increases the length of the root by 23% from 30 to 37 mm, the length of the seedling by 9% from 57 to 62 mm and the increase of plant dry weight by 32%, from 0.19 to 0.25 g (Table 20).

### Example 29

Use of the product to stimulate the carrot plant growth. The experiment was conducted similarly to the Example 28. Carrot seeds of variety 'Krasniy Velikan' were treated with the dilutions of the product. The dry weight of plants was determined after 10 days.

**Table 21**

| Growth parameters of carrot plants when using the biological product "PHENOX" | |
|---|---|
| The variation of treatment | Parameter |
| | dry weight, g |
| The control (water) | 0.03 |
| The control (IBA) | 0.05 |
| PHENOX+BL-5 at the product dilution 1:10 | 0.04 |
| PHENOX+BL-5 at the product dilution 1:100 | 0.04 |
| PHENOX+BL-5 at the product dilution 1:1000 | 0.05 |

The product at the dilution of 1:1000 increased the dry weight of seedlings of carrots by 67% - from 0.03 to 0.05 g (Table 21).

The presence of pesticides in soil, water and industrial wastewater was tested using the gas-chromatic method for the determining of pesticides described by Rudakov O.B. and others (Sputnik khromotografista. - Voronezh: Vodoley, 2004. - p. 528).

### Sources of Information

1. Feodorov A. Yu., Volchenko E.V., Korzhenevich V. I., Singircev I.N., Krestyaninov V.Yu. Polysubstratnyj shtamm-destruktor komponentov stochnih vod proizvodstva fenola. // Prikladnaya biohimiya I mikrobiologiya. - 1993. - I.29, N5. - p.716-722.
2. Feodorov A. Yu., Ignatov O. V., Korzhenevich V. I., Shub G. M. Effect of immobilization in agar gel on the microbial degradation of xenobiotics. //6th European Congr. Biotechnology (ECB6), Firenze, Italy, June 13-17,1993. - Firenze, 1993. - Abstrs. Books, V.2. -TU2I2.
3. Korzhenevich V.I., Volchenko E.V., Singircev I.N., Feodorov A.Yu., Shoob G.M. Microbial treatment of phenolic wastes. - In: Environmental Biotechnol.: Principles and Applications. - M. Moo-Young et al. (eds.), Kluwer Academic Publishers, 1995. -P.498-503.
4. Singircev I.N., Volchenko E.V., Korzhenevich V.I., Gumenyuk A.P., Feodorov A.Yu. Microbal degradation of components of phenolic waste waters. // Prikladnaya biohimiya I mikrobiologiya. - 2000. - I.36, N2. - p.178-188.
5. Authors' certificate No. SU 1597346; C02F3/34, C12N1/20; Strain Pseudomonas pseudoalcaligenes used for treatment of wastes from aromatic compounds /Mironov A.D., Korzhenevich V. I., Barkovskiy A.L. -OG No. 37, 1990.
6. Mironov A.D., Krestyaninov V.Yu., Korzhenevich V. I., Yevtushenko I.Ya., Barkovskiy A.L. Destrukciya 2-nitrobenzoynoy kisloty i drugih aromaticheskih sejedineniy shtammom Pseudomonas pseudoalcaligenes. // Prikladnaya biohimiya I mikrobiologiya. - 1991. - 1.27, N4. - p.571-576.
7. Giardina M.C., Giardi M.T., Filacchione G. Atrazine metabolism by Nocardia: elucidation of the initial pathway and synthesis of potential metabolites. // Agric. Biol. Chem. - 1982. - Vol. 46. - P.1439-1445/
8. Blaszak M., Pelech R., Graczyk P. Screening of Microorganisms for Biodegradation of Simazine Pollution (Obsolete Pesticide Azotop 50 WP) // Water Air Soil Pollut. - 2011. Vol. 220. P. 373-385.
9. Sajjaphan K, Heepngoen P, Sadowsky MJ, Boonkerd N.J Arthrobacter sp. strain KU001 isolated from a Thai soil degrades atrazine in the presence of inorganic nitrogen sources // Microbiol. Biotechnol. - 2010. Vol. 20. - P. 602-608.

## Claims

1. An association of strains of *Pseudomonas putida* deposited in Russian National Collection of Industrial Microorganisms (VKPM) under the accession number B-10997, *Bacillus subtilis* deposited in Russian National Collection of Industrial Microorganisms (VKPM) under the accession number B-10999 and *Rhodococcus erythropolis* deposited in Russian National Collection of Industrial Microorganisms (VKPM) under the accession number Ac-1882.

2. A product containing the association strains of *Pseudomonas putida* VKPM B-10997, *Bacillus subtilis* VKPM B-10999 and *Rhodococcus erythropolis* VKPM Ac-1882.

3. The product according to claim 1 or 2 comprising the association of strains *Pseudomonas putida* VKPM B-10997, *Bacillus subtilis* VKPM B-10999 and *Rhodococcus erythropolis* VKPM Ac-1882 in a weight ratio of (1-2):(1-2):1.

4. The product according to claim 2 or 3 further comprising a sorbent or being immobilized on the sorbent and optionally mineral, organic and/or stimulating additives.

5. Use of the product according to any one of claims 2-4 for the purification of ground, soil, water or industrial wastewater from stable to degradation pesticides, phenol or oil by introduction of the product according to any one of claims 2-4 in an effective amount in contaminated soil, ground, water or wastewater.

6. The use of the product according to claim 5, wherein stable to degradation pesticides are being selected from the group consisting of chlorophenoxy acetic acids, such as 2,4-dichlorophenoxyacetic acid (2,4-D), trichlorophenoxyacetic acid (2,4,5-T), chlorophenoxyacetic acid (CPAA), phenoxyacetic acid (PAA), as well as 2,4-dichlorophenol, phenol, imidacloprid, dichloralurea, tetramethylthiuram disulfide (TMTD).

7. Use of the product according to any one of claims 2-4 for stimulating growth activity of plants and/or as fungicidal preparation.

8. The use of the product according to claim 7, wherein dry composition of the said product is being introduced into the ground during ploughing, tillage or seeding, preferably, in an amount of 10 kg per 1 ha of land.

9. The use of the product according to claim 7, wherein the said product in the form of an aqueous solution is being introduced into the ground by irrigation.

10. The use of the product according to claim 7, wherein introduction of the said product into the soil is carried out by introducing into the soil seeds treated with the suspension of the said product, optionally, with addition of nutrients, such as vermicompost.

## Patentansprüche

1. In der Russischen Nationalsammlung für industrielle Mikroorganismen (VKPM) wurde unter der Zugangsnummer B-10997 eine Gruppe von Stämmen der Pseudomonas putida, unter der Zugangsnummer B-10999 eine Gruppe von Stämmen der Bacillus subtilis und unter der ZugangsnummerAc-1882 eine Gruppe von Stämmen der Rhodococcus erythropolis abgeschieden.

2. Ein Produkt, das die Gruppe der Stämme der Pseudomonas putida, VKPM B-10997, Bacillus subtilis VKPM B-10999 und Rhodococcus erythropolis VKPM Ac-1882 enthält.

3. Ein Produkt gemäß Anspruch 2, das die Gruppe der Stämme von Pseudomonas putida VKPM B-10997, Bacillus subtilis VKPM B-10999 und Rhodococcus erythropolis VKPM Ac-1882, in einem Gewichtsverhältnis von (1-2) :(1-2):1, enthält.

4. Das Produkt gemäß Anspruch 2 oder 3 umfasst außerdem ein Sorptionsmittel oder wird auf dem Sorptionsmittel gegebenenfalls mit mineralischen, organischen und/oder stimulierenden Additiven immobilisiert.

5. Verwendung des Produkts nach einem der Ansprüche 2-4 für die Auswaschung stabiler bis degradierter Pestizide, Phenole oder Öle aus Erde, Boden, Wasser oder Industrieabwasser durch Einbringung des Produkts nach einem der Ansprüche 2-4 in einer wirksamen Menge im kontaminierten Boden, Erdreich, Wasser oder Abwasser.

6. Verwendung des Produkts nach Anspruch 5, bei dem stabile bis degradierte Pestizide aus der Gruppe der Chlorphenoxyessigsäuren, zum Beispiel 2,4-Dichlorofenoxyessigsäure (2,4-D), Trichlorofenoxyessigsäure (2,4, 5-T), Chlor-Fenoxy-Essigsäure (CPAA), Phenoxyessigsäure (PAA) und 2,4-Dichlorphenol, Phenol, Imidacloprid, Dichloralurea, Tetramethylthiuramdisulfid (TMTD) gewählt werden.

7. Verwendung des Produkts nach einem der Ansprüche 2-4, zur Stimulierung der Wachstumsaktivität von Pflanzen und/oder als fungizide Zubereitung.

8. Verwendung des Produktes nach Anspruch 7, wobei die trockene Zusammensetzung des genannten Produkts während des Pflügens, der Bodenbearbeitung oder beim Säen in den Boden, vorzugsweise in einer Menge von 10 kg pro 1 ha, eingearbeitet wird.

9. Verwendung des Produktes nach Anspruch 7, wobei das besagte Produkt in Form einer wässrigen Lösung durch Bewässerung in den Boden eingebracht wird.

10. Verwendung des Produktes nach Anspruch 7, wobei die Einbringung des genannten Produktes in das Erdreich mithilfe von Samen geschieht, der mit der Suspension des genannten Produkts, gegebenenfalls unter Zusatz von Nährstoffen, wie Wurmkompost, behandelt wird.

## Revendications

1. Association de souches de *Pseudomonas putida* déposées à la Russian National Collection of Industrial Microorganisms (VKPM - Collection nationale russe de micro-organismes industriels) sous le numéro d'accès B-10997, de *Bacillus subtilis* déposéees à la Russian National Collection of Industrial Microorganisms (VKPM) sous le numéro d'accès B-10999 et de *Rhodococcus erythropolis* déposées à la Russian National Collection of Industrial Microorganisms (VKPM) sous le numéro d'accès Ac-1882.

2. Produit contenant les souches d'association de *Pseudomonas putida* VKPM B-10997, *Bacillus subtilis* VKPM B-10999 et *Rhodococcus erythropolis* VKPM Ac-1882.

3. Produit selon la revendication 2, comprenant l'association de souches *Pseudomonas putida* VKPM B-10997, *Bacillus subtilis* VKPM B-10999 et *Rhodococcus erythropolis* VKPM Ac-1882 dans un rapport pondéral de (1-2):(1-2):1.

4. Produit selon la revendication 2 ou 3, comprenant en outre un sorbant ou étant immobilisé sur le sorbant et éventuellement des additifs minéraux, organiques et/ou stimulants.

5. Utilisation du produit selon l'une quelconque des revendications 2 à 4, pour la purification de sol, terre, eau ou eaux usées industrielles des pesticides, phénol ou pétrole, stables à la dégradation, par l'introduction du produit selon l'une quelconque des revendications 2 à 4 en une quantité efficace dans de la terre, un sol, de l'eau ou des eaux usées, contaminés.

6. Utilisation du produit selon la revendication 5, dans laquelle les pesticides stables à la dégradation sont choisis dans le groupe constitué d'acides chlorophénoxyacétiques, tels que l'acide 2,4-dichlorophénoxyacétique (2,4-D), l'acide trichlorofénoxyacétique (2,4,5-T), l'acide chlorophénoxyacétique (CPAA), l'acide phénoxyacétique (PAA), ainsi que le 2,4-dichlorophénol, le phénol, l'imidaclopride, la dichloralurée, le disulfure de tétraméthylthiurame (TMTD).

7. Utilisation du produit selon l'une quelconque des revendications 2 à 4, pour stimuler l'activité de croissance de plantes et/ou en tant que préparation fongicide.

8. Utilisation du produit selon la revendication 7, dans laquelle la composition sèche dudit produit est introduite dans le sol pendant le labourage, la culture ou l'ensemencement, de préférence, en une quantité de 10 kg pour 1 hectare de terrain.

9. Utilisation du produit selon la revendication 7, dans laquelle ledit produit sous la forme d'une solution aqueuse est introduit dans le sol par irrigation.

10. Utilisation du produit selon la revendication 7, dans laquelle l'introduction dudit produit dans la terre est effectuée en introduisant dans la terre des semences traitées avec la suspension dudit produit, éventuellement, avec l'addition de nutriments, tels que du lombricompost.


### SEQUENCE LISTING

<110> BIOLAND, Ltd ZAKUTAJEVA, Galina
<120> BIOLOGICAL PRODUCT FOR CLEARING OF WATER, INDUSTRIAL WASTEWATER AND SOIL FROM CHEMICALS, WHICH ARE RESISTANT TO DEGRADATION AND METHOD FOR USING THE SAME
<130> LAP 2/1983
<150> RU2011128996
   <151> 2011-07-13
<160> 3
<170> BiSSAP 1.0
<210> 1
   <211> 1488
   <212> RNA
   <213> Bacillus subtilis
<220>
   <221> source
   <222> 1..1488
   <223> /mol_type="RNA" /organism="Bacillus subtilis"
<400> 1
   ggcucaggac gaacgcuggc ggcgugccua auacaugcaa gucgagcgga cagaugggag      60
   cuugcucccu gauguuagcg gcggacgggu gaguaacacg uggguaaccu gccuguaaga     120
   cugggauaac uccgggaaac cggggcuaau accggauggu uguuugaacc gcaugguuca     180
   aacauaaaag guggcuucgg cuaccacuua cagauggacc cgcggcgcau uagcuaguug     240
   gugagguaac ggcucaccaa ggcaacgaug cguagccgac cugagagggu gaucggccac     300
   acugggacug agacacggcc cagacuccua cgggaggcag caguagggaa ucuuccgcaa     360
   uggacgaaag ucugacggag caacgccgcg ugagugauga agguuuucgg aucguaaagc     420
   ucuguuguua gggaagaaca aguaccguuc gaauagggcg guaccuugac gguaccuaac     480
   cagaaagcca cggcuaacua cgugccagca gccgcgguaa uacguaggug gcaagcguug     540
   uccggaauua uugggcguaa agggcucgca ggcgguuucu uaagucugau gugaaagccc     600
   ccggcucaac cggggagggu cauuggaaac uggggaacuu gagugcagaa gaggagagug     660
   gaauuccacg uguagcggug aaaugcguag agauguggag gaacaccagu ggcgaaggcg     720
   acucucuggu cuguaacuga cgcugaggag cgaaagcgug gggagcgaac aggauuagau     780
   acccugguag uccacgccgu aaacgaugag ugcuaagugu uaggggguuu ccgccccuua     840
   gugcugcagc uaacgcauua agcacuccgc cuggggagua cggucgcaag acugaaacuc     900
   aaaggaauug acgggggccc gcacaagcgg uggagcaugu gguuuaauuc gaagcaacgc     960
   gaagaaccuu accaggucuu gacauccucu gacaauccua gagauaggac guccccuucg    1020
   ggggcagagu gacagguggu gcaugguugu cgucagcucg ugucgugaga uguuggguua    1080
   agucccgcaa cgagcgcaac ccuugaucuu aguugccagc auuuaguugg gcacucuaag    1140
   gugacugccg gugacaaacc ggaggaaggu ggggaugacg ucaaaucauc augccccuua    1200
   ugaccugggc uacacacgug cuacaaugga cagaacaaag ggcagcgaaa ccgcgagguu    1260
   aagccaaucc cacaaaucug uucucaguuc ggaucgcagu cugcaacucg acugcgugaa    1320
   gcuggaaucg cuaguaaucg cggaucagca ugccgcggug aauacguucc cgggccuugu    1380
   acacaccgcc cgucacacca cgagaguuug uaacacccga agucggugag guaaccuuuu    1440
   aggagccagc cgccgaaggu gggacagaug auugggguga agucguaa                 1488
<210> 2
   <211> 1471
   <212> RNA
   <213> Rhodococcus erythropolis
<220>
   <221> source
   <222> 1..1471
   <223> /mol_type="RNA" /organism="Rhodococcus erythropolis"
<400> 2
   uggcucagga cgaacgcugg cggcgugcuu aacacaugca agucgagcgg uaaggccuuu      60
   cgggguacac gagcggcgaa cgggugagua acacgugggu gaucugcccu gcacuucggg     120
   auaagccugg gaaacugggu cuaauaccgg auaugaccuc agguugcaug acuuggggug     180
   gaaagauuua ucggugcagg augggcccgc ggccuaucag cuuguuggug ggguaauggc     240
   cuaccaaggc gacgacgggu agccgaccug agagggugac cggccacacu gggacugaga     300
   cacggcccag acuccuacgg gaggcagcag uggggaauau ugcacaaugg gcgaaagccu     360
   gaugcagcga cgccgcguga gggaugacgg ccuucggguu guaaaccucu uucagcaggg     420
   acgaagcgca agugacggua ccugcagaag aagcaccggc uaacuacgug ccagcagccg     480
   cgguaauacg uagggugcaa gcguuguccg gaauuacugg gcguaaagag uucguaggcg     540
   guuugucgcg ucguuuguga aaaccagcag cucaacugcu ggcuugcagg cgauacgggc     600
   agacuugagu acugcagggg agacuggaau uccuggugua gcggugaaau gcgcagauau     660
   caggaggaac accgguggcg aaggcggguc ucugggcagu aacugacgcu gaggaacgaa     720
   agcgugggua gcgaacagga uuagauaccc ugguaguaca cgccguaaac ggugggcgcu     780
   aggugugggu uccuuccacg gaauccgugc cguagcuaac gcauuaagcg ccccgccugg     840
   ggaguacggc cgcaaggcua aaacucaaag gaauugacgg gggcccgcac aagcggcgga     900
   gcauguggau uaauucgaug caacgcgaag aaccuuaccu ggguuugaca uauaccggaa     960
   agcugcagag auguggcccc ccuugugguc gguauacagg uggugcaugg cugucgucag   1020
   cucgugucgu gagauguugg guuaaguccc gcaacgagcg caaccccuau cuuauguugc    1080
   cagcacguua ugguggggac ucguaagaga cugccggggu caacucggag gaaggugggg    1140
   acgacgucaa gucaucaugc cccuuauguc cagggcuuca cacaugcuac aauggccagu    1200
   acagagggcu gcgagaccgu gagguggagc gaaucccuua aagcuggucu caguucggau    1260
   cggggucugc aacucgaccc cgugaagucg gagucgcuag uaaucgcaga ucagcaacgc    1320
   ugcggugaau acguucccgg gccuuguaca caccgcccgu cacgucauga aagucgguaa    1380
   cacccgaagc cgguggcuua accccuugug ggagggagcc gucgaaggug ggaucggcga    1440
   uugggacgaa gucguaacaa gguagccgua a                                   1471
<210> 3
   <211> 1492
   <212> RNA
   <213> Pseudomonas putida
<220>
   <221> source
   <222> 1..1492
   <223> /mol_type="RNA" /organism="Pseudomonas putida"
<400> 3
   uagaguuuga ucauggcuca gauugaacgc uggcggcagg ccuaacacau gcaagucgag      60
   cggaugacgg gagcuugcuc cuugauucag cggcggacgg gugaguaaug ccuaggaauc     120
   ugccugguag ugggggacaa cguuucgaaa ggaacgcuaa uaccgcauac guccuacggg    180
   agaaagcagg ggaccuucgg gccuugcgcu aucagaugag ccuaggucgg auuagcuagu     240
   ugguggggua auggcucacc aaggcgacga uccguaacug gucugagagg augaucaguc    300
   acacuggaac ugagacacgg uccagacucc uacgggaggc agcagugggg aauauuggac     360
   aaugggcgaa agccugaucc agccaugccg cgugugugaa gaaggucuuc ggauuguaaa     420
   gcacuuuaag uugggaggaa gggcaguaag cuaauaccuu gcuguuuuga cguuaccgac     480
   agaauaagca ccggcuaacu cugugccagc agccgcggua auacagaggg ugcaagcguu     540
   aaucggaauu acugggcgua aagcgcgcgu aggugguuug uuaaguugga ugugaaagcc    600
   ccgggcucaa ccugggaacu gcauccaaaa cuggcaagcu agaguacggu agaggguggu    660
   ggaauuuccu guguagcggu gaaaugcgua gauauaggaa ggaacaccag uggcgaaggc     720
   gaccaccugg acugauacug acacugaggu gcgaaagcgu ggggagcaaa caggauuaga     780
   uacccuggua guccacgccg uaaacgaugu caacuagccg uuggaauccu ugagauuuua     840
   guggcgcagc uaacgcauua aguugaccgc cuggggagua cggccgcaag guuaaaacuc     900
   aaaugaauug acgggggccc gcacaagcgg uggagcaugu gguuuaauuc gaagcaacgc     960
   gaagaaccuu accaggccuu gacaugcaga gaacuuucca gagauggauu ggugccuucg    1020
   ggaacucuga cacaggugcu gcauggcugu cgucagcucg ugucgugaga uguuggguua    1080
   agucccguaa cgagcgcaac ccuuguccuu aguuaccagc acguuauggu gggcacucua    1140
   aggagacugc cggugacaaa ccggaggaag guggggauga cgucaaguca ucauggcccu    1200
   uacggccugg gcuacacacg ugcuacaaug gucgguacag aggguugcca agccgcgagg    1260
   uggagcuaau cucacaaaac cgaucguagu ccggaucgca gucugcaacu cgacugcgug    1320
   aagucggaau cgcuaguaau cgcgaaucag aaugucgcgg ugaauacguu cccgggccuu    1380
   guacacaccg cccgucacac caugggagug gguugcacca gaaguagcua gucuaaccuu    1440
   cgggaggacg guuaccacgg ugugauucau gacuggggug aagucguaac aa           1492